# EUROPÄISCHE PATENTSCHRIFT

(11) **EP 1 523 471 B1**
(45) Veröffentlichungstag und Bekanntmachung des Hinweises auf die Patenterteilung: **16.09.2009**
(21) Anmeldenummer: 03740386.2
(22) Anmeldetag: 30.06.2003
(51) Int. Cl.: C07C 275/54, C07D 239/96, A61K 31/17, A61P 3/10

(54) **HARNSTOFF- UND URETHAN-SUBSTITUIERTE ACYLHARNSTOFFE, VERFAHREN ZU DEREN HERSTELLUNG UND DEREN VERWENDUNG ALS ARZNEIMITTEL**
UREA-SUBSTITUTED AND URETHANE-SUBSTITUTED ACYLUREAS, METHODS FOR THE PRODUCTION THEREOF AND THEIR USE AS MEDICAMENTS
ACYLUREES SUBSTITUEES PAR UREE ET URETHANE, PROCEDES PERMETTANT DE LES PRODUIRE ET LEUR UTILISATION COMME MEDICAMENTS

(30) Priorität: 11.07.2002 DE 10231371
(43) Veröffentlichungstag der Anmeldung: 20.04.2005
(73) Patentinhaber: Sanofi-Aventis Deutschland GmbH, 65929 Frankfurt am Main (DE)
(72) Erfinder: DEFOSSA, Elisabeth, 65510 Idstein (DE); KADEREIT, Dieter, 63071 Offenbach (DE); KLABUNDE, Thomas, 65929 Frankfurt (DE); BURGER, Hans-Joerg, Morristown, NJ 07960 (US); HERLING, Andreas, 65520 Bad Camberg (DE); WENDT, Karl-Ulrich, 60487 Frankfurt (DE); VON ROEDERN, Erich, 65795 Hattersheim (DE); SCHOENAFINGER, Karl, 63755 Alzenau (DE)
(86) Internationale Anmeldenummer: PCT/EP2003/006934
(87) Internationale Veröffentlichungsnummer: WO 2004/007437

(56) Entgegenhaltungen:
- EP-A- 0 116 729
- EP-A- 0 167 197
- EP-A- 0 221 847
- WO-A-01/94300
- WO-A-02/096864
- PATENT ABSTRACTS OF JAPAN vol. 013, no. 224 (C-599), 24. Mai 1989 (1989-05-24) & JP 01 034953 A (AGURO KANESHO KK), 6. Februar 1989 (1989-02-06)

## Beschreibung

Die Erfindung betrifft Harnstoff- und Urethan-substituierte Acylharnstoffe sowie deren physiologisch verträgliche Salze und physiologisch funktionellen Derivate.

In EP 0 221 847 sind strukturähnliche Verbindungen zur Schädlingsbekämpfung beschrieben.
In WO 02/096864 sind strukturähnliche Verbindungen zur Behandlung von Diabetes beschrieben.
In WO 01/94300 sind strukturähnliche Verbindungen zur Behandlung von Diabetes beschrieben.
Patent Abstracts of Japan bd. 013, Nr. 224, (C-599), 24. Mai 1989 & JP 01 034953 A, 6. Februar 1989 beschreibt strukturähnliche Verbindungen.

Der Erfindung lag die Aufgabe zugrunde, Verbindungen zur Verfügung zu stellen, mit denen eine Prävention und Behandlung von Diabetes Typ 2 möglich ist. Die Verbindungen sollen dazu eine merkliche Senkung des Blutzuckerspiegels bewirken.

Die Erfindung betrifft daher Verbindungen der Formel Ia, 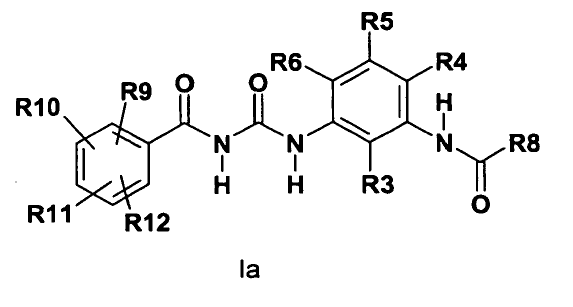 worin bedeuten
- R9, R10, R11: unabhängig von einander F oder Cl;
- R12: H;
- R13: unabhängig voneinander H oder (C₁-C₆)-Alkyl;
- R1, R2: H;
- R3, R4, R5: unabhängig voneinander H, COOR13;
- R6: Cl, OCF₃, COOR13, N(R14)(R15), (C₂-C₆)-Alkenyl, O-(C₁-C₁₀)-Alkyl, wobei Alkyl, Alkenyl mehrfach mit F, COOR13 oder CON(R14)(R15) substituiert sein können;
- R14, R15: (C₁-C₆)-Alkyl, wobei Alkyl mit N(R13)₂ substituiert sein kann;
oder die Reste R14 und R15 mit dem Stickstoffatom an das sie gebunden sind einem 5 gliedrigen, gesättigten heterocyclischen Ring bilden;
- R8: N(R18)(R19) oder OR20;
- R18, R19: unabhängig voneinander H, (C₁-C₁₀)-Alkyl, (C₂-C₁₀)-Alkenyl, wobei Alkyl durch COOR13, N(R13)₂ oder Phenyl substituiert sein kann, (C₃-C₇)-Cycloalkyl oder (C₆-C₁₀)-Aryl, wobei Aryl mehrfach mit F, Cl, CN, (C₁-C₆)-Alkyl, O-(C₁-C₆)-Alkyl, CO-(C₁-C₆)-Alkyl, wobei Alkyl mehrfach durch F substituiert sein kann, O-Phenyl, Phenyl, Pyridyl oder COOR13 substituiert sein kann;
- R20: (C₁-C₁₀)-Alkyl, (C₂-C₁₀)-Alkenyl, (C₂-C₁₀)-Alkinyl oder Phenyl, wobei Phenyl mit Cl oder (C₁-C₆)-Alkyl substituiert sein kann;
sowie deren physiologisch verträgliche Salze.

Die Alkylreste in den Substituenten R1, R2, R3, R4, R5, R6, R7, R8, R9, R10, R11 R12, R13, R14, R15, R16, R17, R18, R19 oder R20 können sowohl geradkettig wie verzweigt sein.

Können Reste oder Substituenten mehrfach in den Verbindungen der Formel I auftreten, wie zum Beispiel COOR13, so können sie alle unabhängig voneinander die angegebene Bedeutungen haben und gleich oder verschieden sein.

Die Erfindung bezieht sich auf Verbindungen der Formel I, in Form ihrer Racemate, racemischen Mischungen und reinen Enantiomere sowie auf ihre Diastereomere und Mischungen davon.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), und Salze von Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel 1, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel" auf Verbindung(en) der Formel I wie vorstehend beschrieben, sowie ihre Salze, Solvate und physiologisch funktionellen Derivate wie hierin beschrieben.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-ÖI-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, GLP-1-Agonisten,
Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit einem PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US00/11833, PCT/US00/11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit einem Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z. B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al. in: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) , Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1 H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

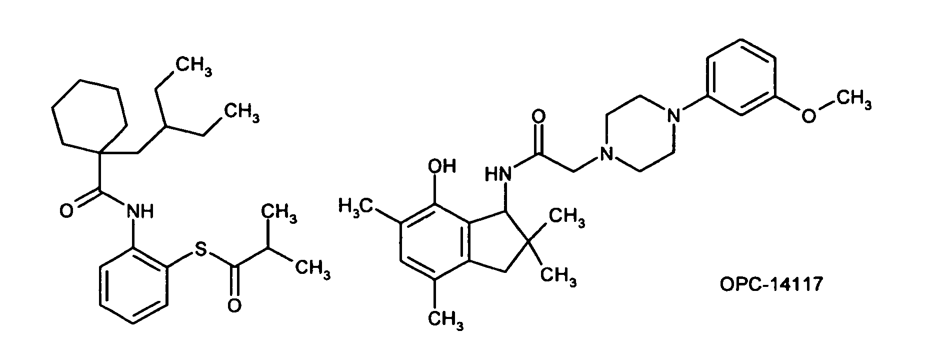

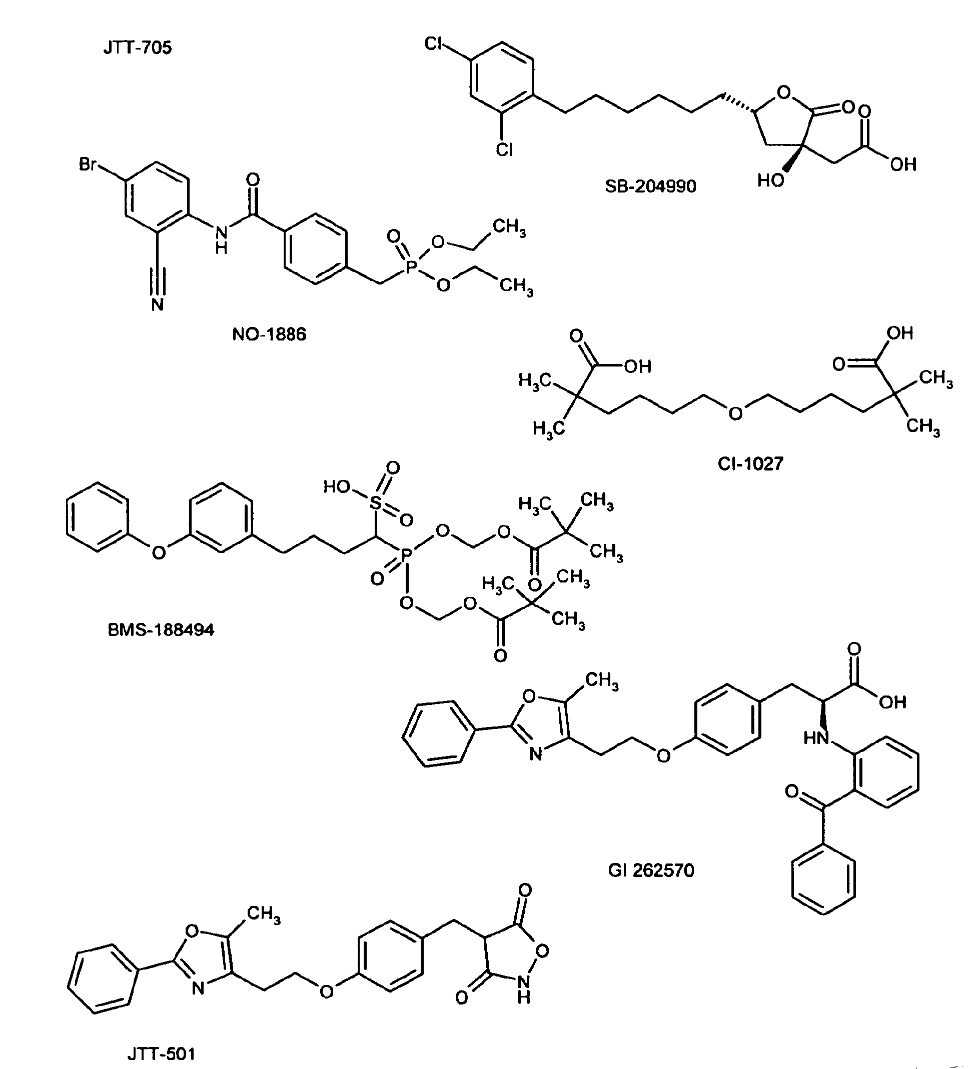

Die nachfolgend aufgeführten Beispiele dienen zur Erläuterung der Erfindung.

**Tabelle 1: Beispiele der Formel I**

| | | | | | | | | |
|---|---|---|---|---|---|---|---|---|
| 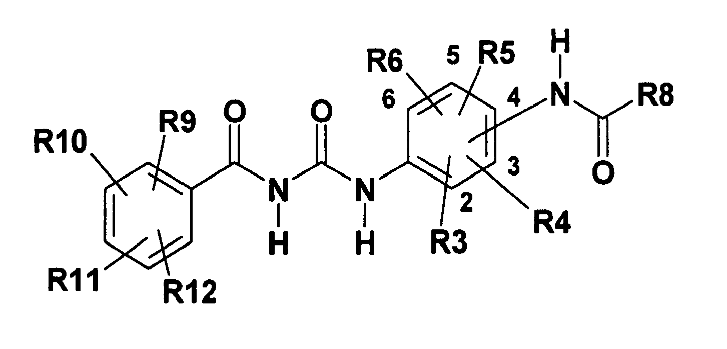 | | | | | | | | |

| **Bsp.** | **R9, R10, R11, R12** | **R3** | **R4** | **R5** | **R6** | **Verkn.** | **R8** | **MS *** |
|---|---|---|---|---|---|---|---|---|
| **1** | 2-Cl-4,5-F₂ | 2-H | 4-H | 5-H | 6-OCH₃ | 3 | NHCH₃ | ok |
| **2** | 2-Cl-4,5-F₂ | 2-H | 4-COOC₂H₅ | 5-H | 6-OCH₃ | 3 | NHCH₃ | ok |
| **3** | 2-Cl-4-F | 2-H | 3-COOH | 4-H | 5-H | 6 | OCH₃ | ok |
| **4** | 2-Cl-4,5-F₂ | 2-H | 4-H | 5-H | 6-OCF₃ | 3 | NHC₂H₅ | ok |
| **5** | 2-Cl-4,5-F₂ | 2-H | 4-H | 5-H | 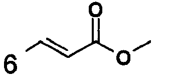 | 3 | NHC₂H₅ | ok |
| **6** | 2-Cl-4,5-F₂ | 2-H | 4-H | 5-H | 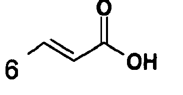 | 3 | NHC₂H₅ | ok |
| **7** | 2-Cl-4,5-F₂ | 2-H | 4-H | 5-H | 6-N(CH₃)₂ | 3 | NHC₂H₅ | ok |
| **8** | 2-Cl-4,5-F₂ | 2-H | 4-H | 5-H | 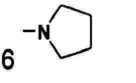 | 3 | NHC₂H₅ | ok |
| **9** | 2-Cl-4,5-F₂ | 2-H | 4-H | 5-H | 6-Cl | 3 | NHC₂H₅ | ok |
| **10** | 2-Cl-4,5-F₂ | 2-H | 4-H | 5-H | 6-OCH₃ | 3 | NHC₂H₅ | ok |
| **11** | 2-Cl-4,5-F₂ | 2-H | 4-COOC₂H₅ | 5-H | 6-OCH₃ | 3 | NHC₂H₅ | ok |
| **12** | 2-Cl-4,5-F₂ | 2-H | 4-H | 5-H | 6-OCH₃ | 3 | 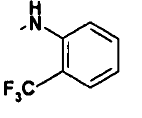 | ok |
| **13** | 2-Cl-4,5-F₂ | 2-H | 4-H | 5-H | 6-OCH₃ | 3 | 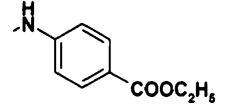 | ok |
| **14** | 2-Cl-4,5-F₂ | 2-H | 4-H | 5-H | 6-OCH₃ | 3 | 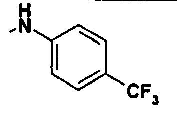 | ok |
| **15** | 2-Cl-4, 5-F₂ | 2-H | 4-H | 5-H | 6-OCH₃ | 3 | 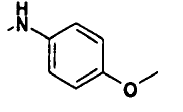 | ok |
| **16** | 2-Cl-4,5-F₂ | 2-H | 4-H | 5-H | 6-OCH₃ | 3 | 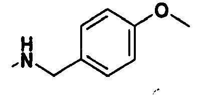 | ok |
| **17** | 2-Cl-4,5-F₂ | 2-H | 4-H | 5-H | 6-OCH₃ | 3 | 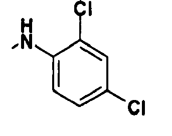 | ok |
| **18** | 2-Cl-4,5-F₂ | 2-H | 4-H | 5-H | 6-OCH₃ | 3 | NHCH₂COOC₂H₅ | ok |
| **19** | 2-Cl-4,5-F₂ | 2-H | 4-H | 5-H | 6-OCH₃ | 3 | 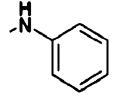 | ok |
| **20** | 2-Cl-4,5-F₂ | 2-H | 4-H | 5-H | 6-OCH₃ | 3 | 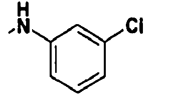 | ok |
| **21** | 2-Cl-4,5-F₂ | 2-H | 4-H | 5-H | 6-OCH₃ | 3 | 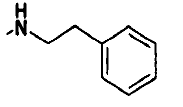 | ok |
| **22** | 2-Cl-4,5-F₂ | 2-H | 4-H | 5-H | 6-OCH₃ | 3 | 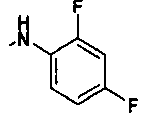 | ok |
| **23** | 2-Cl-4,5-F₂ | 2-H | 4-H | 5-H | 6-OCH₃ | 3 | 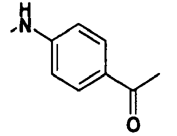 | ok |
| **24** | 2-Cl-4,5-F₂ | 2-H | 4-H | 5-H | 6-OCH₃ | 3 | 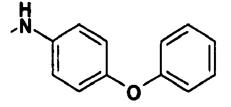 | ok |
| **25** | 2-Cl-4,5-F₂ | 2-H | 4-H | 5-H | 6-OCH₃ | 3 | 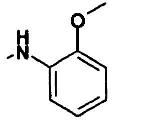 | ok |
| **26** | 2-Cl-4,5-F₂ | 2-H | 4-H | 5-H | 6-OCH₃ | 3 | 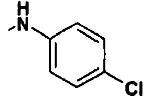 | ok |
| **27** | 2-Cl-4,5-F₂ | 2-H | 4-H | 5-H | 6-OCH₃ | 3 | NH(CH₂)₃CH₃ | ok |
| **28** | 2-Cl-4,5-F₂ | 2-H | 4-H | 5-H | 6-OCH₃ | 3 | 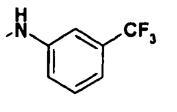 | ok |
| **29** | 2-Cl-4,5-F₂ | 2-H | 4-H | 5-H | 6-OCH₃ | 3 | 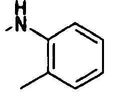 | ok |
| **30** | 2-Cl-4,5-F₂ | 2-H | 4-H | 5-H | 6-OCH₃ | 3 | NHCH(CH₃)₂ | ok |
| **31** | 2-Cl-4,5-F₂ | 2-H | 4-H | 5-H | 6-OCH₃ | 3 | NH(CH₂)₅CH₃ | ok |
| **32** | 2-Cl-4,5-F₂ | 2-H | 4-H | 5-H | 6-OCH₃ | 3 | 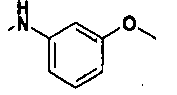 | ok |
| **33** | 2-Cl-4,5-F₂ | 2-H | 4-H | 5-H | 6-OCH₃ | 3 | 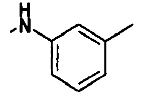 | ok |
| **34** | 2-Cl-4,5-F₂ | 2-H | 4-H | 5-H | 6-OCH₃ | 3 | 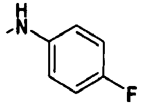 | ok |
| **35** | 2-Cl-4,5-F₂ | 2-H | 4-H | 5-H | 6-OCH₃ | 3 | NH(CH₂)₂CH₃ | ok |
| **36** | 2-Cl-4,5-F₂ | 2-H | 4-H | 5-H | 6-OCH₃ | 3 | 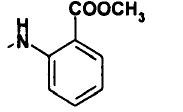 | ok |
| **37** | 2-Cl-4,5-F₂ | 2-H | 4-H | 5-H | 6-OCH₃ | 3 | 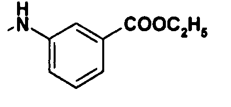 | ok |
| **38** | 2-Cl-4,5-F₂ | 2-H | 4-H | 5-H | 6-OCH₃ | 3 | 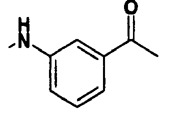 | ok |
| **39** | 2-Cl-4,5-F₂ | 2-H | 4-H | 5-H | 6-OCH₃ | 3 | 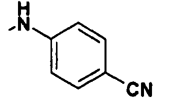 | ok |
| **40** | 2-Cl-4,5-F₂ | 2-H | 4-H | 5-H | 6-OCH₃ | 3 | 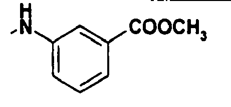 | ok |
| **41** | 2-Cl-4,5-F₂ | 2-H | 4-H | 5-H | 6-OCH₃ | 3 | 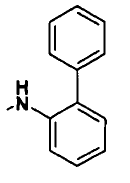 | ok |
| **42** | 2-Cl-4,5-F₂ | 2-H | 4-H | 5-H | 6-OCH₃ | 3 | 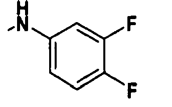 | ok |
| **43** | 2-Cl-4,5-F₂ | 2-H | 4-H | 5-H | 21 6-OCH₃ | 3 | NH(CH₂)₄CH₃ | ok |
| **44** | 2-Cl-4,5-F₂ | 2-H | 4-H | 5-H | 6-OCH₃ | 3 | 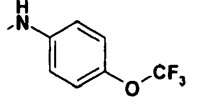 | ok |
| **45** | 2-Cl-4,5-F₂ | 2-H | 4-H | 5-H | 6-OCH₃ | 3 | 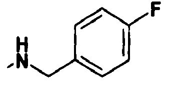 | ok |
| **46** | 2-Cl-4,5-F₂ | 2-H | 4-H | 5-H | 6-OCH₃ | 3 | 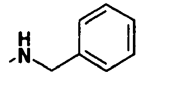 | ok |
| **47** | 2-Cl-4,5-F₂ | 2-H | 4-H | 5-H | 6-OCH₃ | 3 | 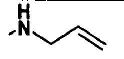 | ok |
| **48** | 2-Cl-4,5-F₂ | 2-H | 4-H | 5-H | 6-OCH₃ | 3 | NH(CH₂)₂COOC₂H₅ | ok |
| **49** | 2-Cl-4,5-F₂ | 2-H | 4-H | 5-H | 6-OCH₃ | 3 | 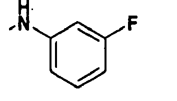 | ok |
| **50** | 2-Cl-4,5-F₂ | 2-H | 4-H | 5-H | 6-OCH₃ | 3 | 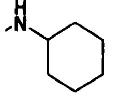 | ok |
| **51** | 2-Cl-4,5-F₂ | 2-H | 4-H | 5-H | 6-OCH₃ | 3 | 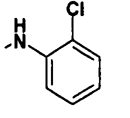 | ok |
| **52** | 2-Cl-4,5-F₂ | 2-H | 4-H | 5-H | 6-OCH₃ | 3 | 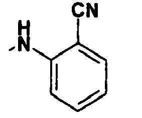 | ok |
| **53** | 2-Cl-4,5-F₂ | 2-H | 4-H | 5-H | 6-OCH₃ | 3 | 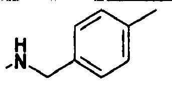 | ok |
| **54** | 2-Cl-4,5-F₂ | 2-H | 4-H | 5-H | 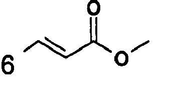 | 3 | 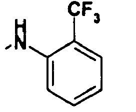 | ok |
| **55** | 2-Cl-4,5-F₂ | 2-H | 4-H | 5-H | 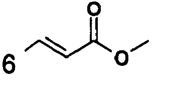 | 3 | 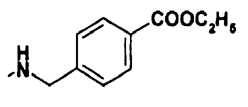 | ok |
| **56** | 2-Cl-4,5-F₂ | 2-H | 4-H | 5-H | 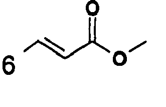 | 3 | 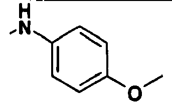 | ok |
| **57** | 2-Cl-4,5-F₂ | 2-H | 4-H | 5-H | 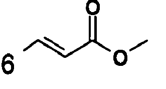 | 3 | 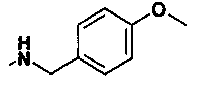 | ok |
| **58** | 2-Cl-4,5-F₂ | 2-H | 4-H | 5-H | 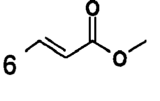 | 3 | 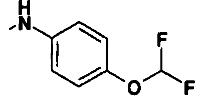 | ok |
| **59** | 2-Cl-4,5-F₂ | 2-H | 4-H | 5-H | 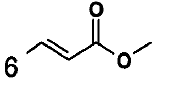 | 3 | NHCH₂COOC₂H₅ | ok |
| **60** | 2-Cl-4,5-F₂ | 2-H | 4-H | 5-H | 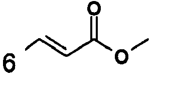 | 3 | 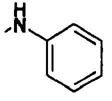 | ok |
| **61** | 2-Cl-4,5-F₂ | 2-H | 4-H | 5-H | 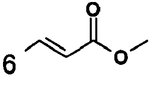 | 3 | 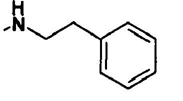 | ok |
| **62** | 2-Cl-4,5-F₂ | 2-H | 4-H | 5-H | 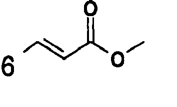 | 3 | 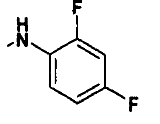 | ok |
| **63** | 2-Cl-4,5-F₂ | 2-H | 4-H | 5-H | 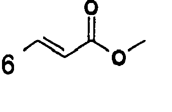 | 3 | 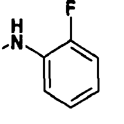 | ok |
| **64** | 2-Cl-4,5-F₂ | 2-H | 4-H | 5-H | 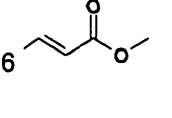 | 3 | 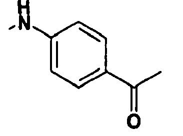 | ok |
| **65** | 2-Cl-4,5-F₂ | 2-H | 4-H | 5-H | 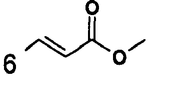 | 3 | 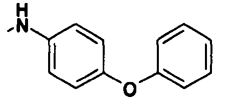 | ok |
| **66** | 2-Cl-4,5-F₂ | 2-H | 4-H | 5-H | 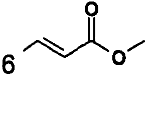 | 3 | 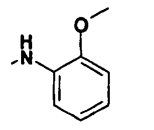 | ok |
| **67** | 2-Cl-4,5-F₂ | 2-H | 4-H | 5-H | 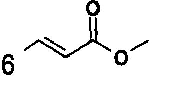 | 3 | 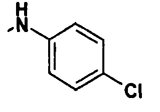 | ok |
| **68** | 2-Cl-4,5-F₂ | 2-H | 4-H | 5-H | 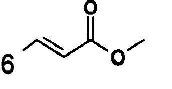 | 3 | NH(CH₂)₃CH₃ | ok |
| **69** | 2-Cl-4,5-F₂ | 2-H | 4-H | 5-H | 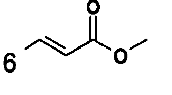 | 3 | 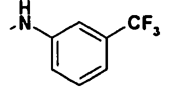 | ok |
| **70** | 2-Cl-4,5-F₂ | 2-H | 4-H | 5-H | 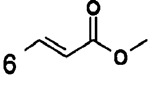 | 3 | 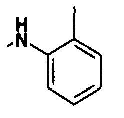 | ok |
| **71** | 2-Cl-4,5-F₂ | 2-H | 4-H | 5-H | 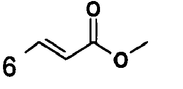 | 3 | NHCH(CH₃)₂ | ok |
| **72** | 2-Cl-4,5-F₂ | 2-H | 4-H | 5-H | 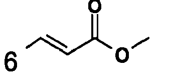 | 3 | NH(CH₂)₅CH₃ | ok |
| **73** | 2-Cl-4,5-F₂ | 2-H | 4-H | 5-H | 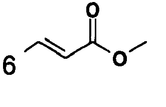 | 3 | 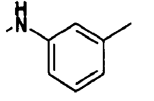 | ok |
| **74** | 2-Cl-4,5-F₂ | 2-H | 4-H | 5-H | 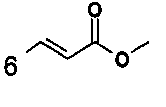 | 3 | 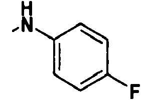 | ok |
| **75** | 2-Cl-4,5-F₂ | 2-H | 4-H | 5-H | 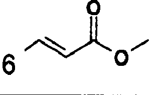 | 3 | NH(CH₂)₂CH₃ | ok |
| **76** | 2-Cl-4,5-F₂ | 2-H | 4-H | 5-H | 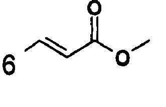 | 3 | 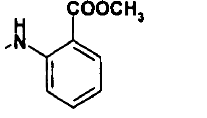 | ok |
| **77** | 2-Cl-4,5-F₂ | 2-H | 4-H | 5-H | 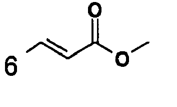 | 3 | 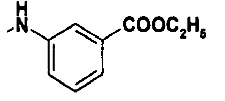 | ok |
| **78** | 2-Cl-4,5-F₂ | 2-H | 4-H | 5-H | 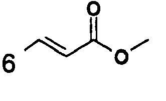 | 3 | 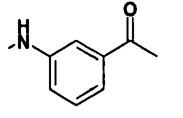 | ok |
| **79** | 2-Cl-4,5-F₂ | 2-H | 4-H | 5-H | 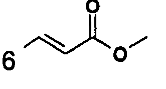 | 3 | 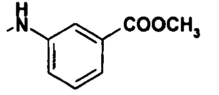 | ok |
| **80** | 2-Cl-4,5-F₂ | 2-H | 4-H | 5-H | 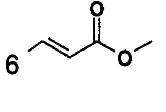 | 3 | 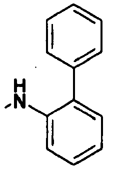 | ok |
| **81** | 2-Cl-4,5-F₂ | 2-H | 4-H | 5-H | 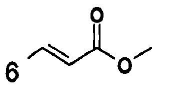 | 3 | 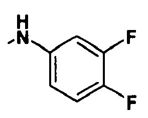 | ok |
| **82** | 2-Cl-4,5-F₂ | 2-H | 4-H | 5-H | 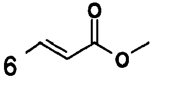 | 3 | 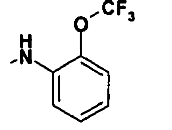 | ok |
| **83** | 2-Cl-4,5-F₂ | 2-H | 4-H | 5-H | 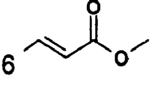 | 3 | NH(CH₂)₄CH₃ | ok |
| **84** | 2-Cl-4,5-F₂ | 2-H | 4-H | 5-H | 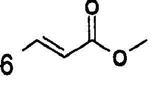 | 3 | 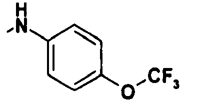 | ok |
| **85** | 2-Cl-4,5-F₂ | 2-H | 4-H | 5-H | 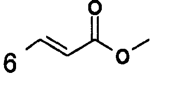 | 3 | 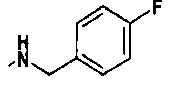 | ok |
| **86** | 2-Cl-4,5-F₂ | 2-H | 4-H | 5-H | 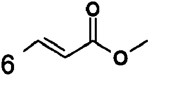 | 3 | 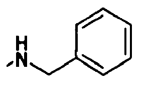 | ok |
| **87** | 2-Cl-4,5-F₂ | 2-H | 4-H | 5-H | 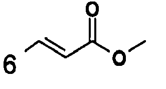 | 3 | 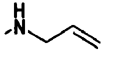 | ok |
| **88** | 2-Cl-4,5-F₂ | 2-H | 4-H | 5-H | 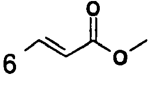 | 3 | NH(CH₂)₂COOC₂H₅ | ok |
| **89** | 2-Cl-4,5-F₂ | 2-H | 4-H | 5-H | 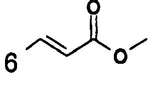 | 3 | 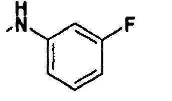 | ok |
| **90** | 2-Cl-4,5-F₂ | 2-H | 4-H | 5-H | 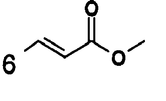 | 3 | 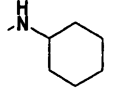 | ok |
| **91** | 2-Cl-4,5-F₂ | 2-H | 4-H | 5-H | 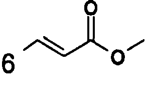 | 3 | 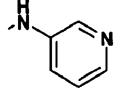 | ok |
| **92** | 2-Cl-4,5-F₂ | 2-H | 4-H | 5-H | 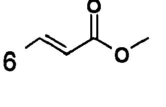 | 3 | 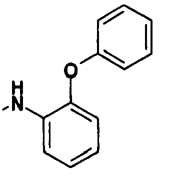 | ok |
| **93** | 2-Cl-4,5-F₂ | 2-H | 4-H | 5-H | 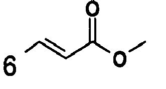 | 3 | 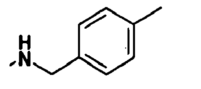 | ok |
| **94** | 2-Cl-4,5-F₂ | 2-H | 4-H | 5-H | 6-OCHF₂ | 3 | OCH₃ | ok |
| **95** | 2-Cl-4,5-F₂ | 2-H | 4-H | 5-H | 6-OCHF₂ | 3 | NHC₂H₅ | ok |
| **96** | 2-Cl-4,5-F₂ | 2-H | 4-H | 5-H | 6-OCH₃ | 3 | O(CH₂)₂CH₃ | ok |
| **97** | 2-Cl-4,5-F₂ | 2-H | 4-H | 5-H | 6-OCH₃ | 3 | 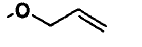 | ok |
| **98** | 2-Cl-4,5-F₂ | 2-H | 4-H | 5-H | 6-OCH₃ | 3 | O(CH₂)₃CH₃ | ok |
| **99** | 2-Cl-4,5-F₂ | 2-H | 4-H | 5-H | 6-OCH₃ | 3 | OCH₂CH(CH₃)₂ | ok |
| **100** | 2-Cl-4,5-F₂ | 2-H | 4-H | 5-H | 6-OCH₃ | 3 | OC₂H₅ | ok |
| **101** | 2-Cl-4,5-F₂ | 2-H | 4-H | 5-H | 6-OCH₃ | 3 | OCH₂C≡CCH₃ | ok |
| **102** | 2-Cl-4,5-F₂ | 2-H | 4-H | 5-H | 6-OCH₃ | 3 | 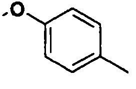 | ok |
| **103** | 2-Cl-4,5-F₂ | 2-H | 4-H | 5-H | 6-OCH₃ | 3 | O(CH₂)₂CH=CH₂ | ok |
| **104** | 2-Cl-4,5-F₂ | 2-H | 4-H | 5-H | 6-OCH₃ | 3 | O(CH₂)₅CH₃ | ok |
| **105** | 2-Cl-4,5₋F₂ | 2-H | 4-H | 5-H | 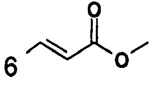 | 3 | O(CH₂)₂CH₃ | ok |
| **106** | 2-Cl-4,5-F₂ | 2-H | 4-H | 5-H | 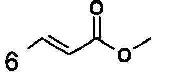 | 3 | 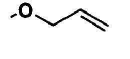 | ok |
| **107** | 2-Cl-4,5-F₂ | 2-H | 4-H | 5-H | 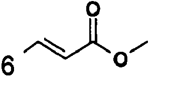 | 3 | O(CH₂)₃CH₃ | ok |
| **108** | 2-Cl-4,5-F₂ | 2-H | 4-H | 5-H | 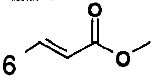 | 3 | OCH₂CH(CH₃)₂ | ok |
| **109** | 2-Cl-4,5-F₂ | 2-H | 4-H | 5-H | 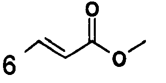 | 3 | OC₂H₅ | ok |
| **110** | 2-Cl-4,5-F₂ | 2-H | 4-H | 5-H | 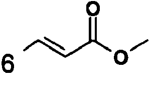 | 3 | 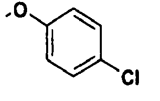 | ok |
| **111** | 2-Cl-4,5-F₂ | 2-H | 4-H | 5-H | 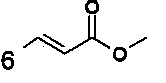 | 3 | OCH₂C≡CCH₃ | ok |
| **112** | 2-Cl-4,5-F₂ | 2-H | 4-H | 5-H | 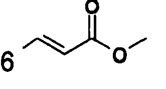 | 3 | 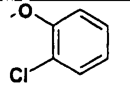 | ok |
| **113** | 2-Cl-4,5-F₂ | 2-H | 4-H | 5-H | 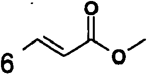 | 3 | O(CH₂)₂CH=CH₂ | ok |
| **114** | 2-Cl-4,5-F₂ | 2-H | 4-H | 5-H | 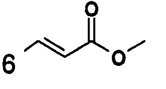 | 3 | O(CH₂)₅CH₃ | ok |
| **115** | 2-Cl-4,5-F₂ | 2-H | 4-H | 5-H | 6-OC₂H₅ | 3 | OCH₃ | ok |
| **116** | 2-Cl-4,5-F₂ | 2-H | 4-H | 5-H | 6-OC₂H₅ | 3 | NHC₂H₅ | ok |
| **117** | 2-Cl-4,5-F₂ | 2-H | 4-H | 5-H | 6-O(CH₂)₂CH₃ | 3 | OCH₃ | ok |
| **118** | 2-Cl-4,5-F₂ | 2-H | 4-H | 5-H | 6-O(CH₂)₂CH₃ | 3 | NHC₂H₅ | ok |
| **119** | 2-Cl-4,5-F₂ | 2-H | 4-H | 5-H | 6-O(CH₂)₃CH₃ | 3 | OCH₃ | ok |
| **120** | 2-Cl-4,5-F₂ | 2-H | 4-H | 5-H | 6-O(CH₂)₃CH₃ | 3 | NHC₂H₅ | ok |
| **121** | 2-Cl-4,5-F₂ | 2-H | 4-H | 5-H | 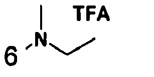 | 3 | OCH₃ | ok |
| **122** | 2-Cl-4,5-F₂ | 2-H | 4-H | 5-H | 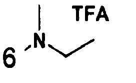 | 3 | NHC₂H₅ | ok |
| **123** | 2-Cl-4,5-F₂ | 2-H | 4-H | 5-H | 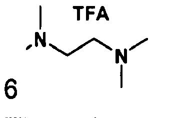 | 3 | OCH₃ | ok |
| **124** | 2-Cl-4,5-F₂ | 2-H | 4-H | 5-H | 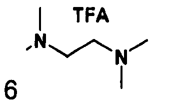 | 3 | NHC₂H₅ | ok |
| **125** | 2,4-Cl₂ | 2-H | 4-H | 5-H | 6-H | 3 | NHCH₃ | ok |
| **126** | 2,4-Cl₂ | 2-H | 4-H | 5-H | 6-Cl | 3 | NHCH₃ | ok |
| **127** | 2,4-Cl₂ | 2-H | 4-H | 5-H | 6-OCH₃ | 3 | NHCH₃ | ok |
| **128** | 2,4-Cl₂ | 2-H | 4-H | 5-H | 6-OCH₃ | 3 | NH(CH₂)₂COOC₂H₅ | ok |
| **129** | 2-Cl-4,5-F₂ | 2-H | 4-H | 5-H | 6-Cl | 3 | NHCH₃ | ok |
| **130** | 2,4-Cl₂ | 2-H | 3-H | 5-H | 6-H | 4 | NHCH₃ | ok |
| **131** | 2,4-Cl₂ | 2-H | 3-H | 5-H | 6-H | 4 | NH(CH₂)₃NH₂ x TFA | ok |
| **132** | 2-Cl-4,5-F₂ | 2-H - | | 5-H | 6-OCH₃ | 3 | 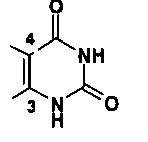 | ok |

| | | | | | | | | |
|---|---|---|---|---|---|---|---|---|
| * Unter der Angabe "MS ist ok" wird verstanden, dass ein Massenspektrum oder HPLC/MS gemessen wurde und in diesem der Molpeak (Molmasse + H⁺) nachgewiesen wurde. | | | | | | | | |

Die Verbindungen der Formel I zeichnen sich durch günstige Wirkungen auf den Zuckerstoffwechsel aus, sie senken insbesondere den Blutzuckerspiegel und sind zur Behandlung von Typ 2 Diabetes geeignet. Die Verbindungen können daher allein oder in Kombination mit weiteren Blutzucker senkenden Wirkstoffen (Antidiabetika) eingesetzt werden.
Die Verbindungen der Formel I eignen sich weiterhin zur Behandlung von Diabetischen Spätschäden, wie z.B. Nephropathie, Retinopathie, Neuropathie sowie Herzinfarkt, Myocardialem Infarkt, peripheren arteriellen Verschlusskrankheiten, Thrombosen, Arteriosklerose, Syndrom X, Obesitas, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten.

Die Wirksamkeit der Verbindungen wurde wie folgt getestet:

### Glykogenphosphorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde.
Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCl, 2,5 mM EDTA, 2,5 mM MgCl₂6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben. Dieser Test wurde mit einer Konzentration von 10 µM der Prüfsubstanz durchgeführt, um die jeweilige Hemmung der Glykogenphosphorylase a in vitro durch die Prüfsubstanz zu bestimmen.

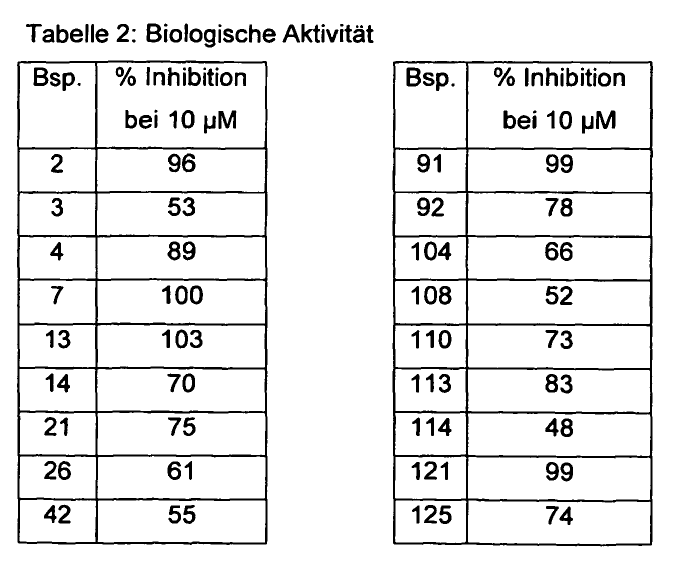

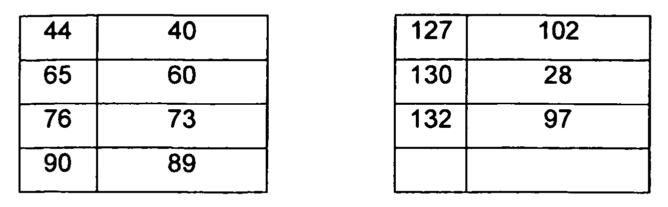

Aus der Tabelle ist abzulesen, dass die Verbindungen der Formel I die Aktivität der Glykogenphosphorylase a hemmen und dadurch zur Senkung des Blutzuckerspiegels gut geeignet sind. Sie eignen sich damit insbesonders zur Prävention und Behandlung von Diabetes Typ 2.

Nachfolgend wird die Herstellung einiger Beispiele detailliert beschrieben, die übrigen Verbindungen der Formel I wurden analog erhalten:

### Experimenteller Teil:

### Beispiel 1:

### 1-{3-[3-(2-Chloro-4,5-difluoro-benzoyl)-ureido]-4-methoxy-phenyl}-3-methyl-harnstoff

### a) 2-Chlor-4,5-difluorbenzoylisocyanat

2-Chlor-4,5-difluorbenzamid wurde in Dichlormethan gelöst, mit 1,5 eq. Oxalylchlorid versetzt und 16 Stunden zum Rückfluss erhitzt. Das Reaktionsgemisch wurde am Hochvakuum eingeengt und ohne weitere Reinigung in Stufe b eingesetzt.

### b) 1-(2-Chloro-4,5-difluoro-benzoyl)-3-(2-methoxy-5-nitro-phenyl)-harnstoff

4,0 g (23,8 mmol) 2-Methoxy-5-nitroanilin wurden in 10 ml N-Methyl-2-pyrrolidinon gelöst und mit 5,2 g (23,8 mmol) 2-Chlor-4,5-difluorbenzoylisocyanat versetzt. Es trat eine leichte Erwärmung auf. Nach 15 Minuten bei Raumtemperatur wurde Diethylether zugegeben und der entstandene Niederschlag abgesaugt. Man erhielt 6,6 g (79%) des gewünschten Produktes.

### c) 1-(5-Amino-2-methoxy-phenyl)-3-(2-chloro-4,5-difluoro-benzoyl)-harnstoff

2,0 g (5,2 mmol) 1-(2-Chloro-4,5-difluoro-benzoyl)-3-(2-methoxy-5-nitro-phenyl)-harnstoff wurden in 20 ml Essigsäureethylester/Methanol-Gemisch bei 70 °C mit 5,8 g (25,9 mmol) Zinndichlorid Hydrat versetzt. Nach 1 Stunde wurden 30 ml N-Methyl-2-pyrrolidinon zugegeben und weitere 2 Stunden gerührt. Nach dem Abkühlen wurde das Reaktionsgemisch basisch gestellt und der entstandene Niederschlag abgesaugt. Die Phasen wurden getrennt. Anschließend wurde die organische Phase dreimal mit Wasser gewaschen, getrocknet und im Hochvakuum eingeengt. Man erhielt 1,2 g (67 %) des gewünschten Produktes.

### d) 1-{3-[3-(2-Chloro-4,5-difluoro-benzoyl)-ureido]-4-methoxy-phenyl}-3-methyl-harnstoff

600 mg (1,7 mmol) 1-(5-Amino-2-methoxy-phenyl)-3-(2-chloro-4,5-difluoro-benzoyl)-harnstoff wurden in 5 ml Acetonitril gelöst und mit 69 mg (1,7 mmol) Methylisocyanat versetzt. Nach einer Stunde Rühren bei Raumtemperatur wurde der entstandenen Niederschlag abgesaugt. Man erhielt 638 mg (91 %) des gewünschten Produktes.

### Beispiel 3:

### 3-[3-(2-Chloro-4-fluoro-benzoyl)-ureido]-4-methoxycarbonylamino-benzoesäure

### a) 2-Chlor-4-fluorbenzoylisocyanat

1,64 g (6 mmol) 2-Chlor-4-fluorbenzamid wurden in 3 ml Dichlormethan gelöst, bei 0 °C und unter Stickstoffatmosphäre mit 0,8 ml (9,3 mmol) Oxalylchlorid versetzt und 9 Stunden zum Rückfluss erhitzt. Das Reaktionsgemisch wurde am Hochvakuum eingeengt und ergab 1,17 g (5,8 mmol) des gewünschten Produktes das als Lösung in Dichlormethan (1 mmol in 1,7 ml Lösung) in Stufe b eingesetzt wurde.

### b) 4-Amino-3-[3-(2-chloro-4-fluoro-benzoyl)-ureido]-benzoesäure

150 mg (1 mmol) 3,4-Diaminobenzoesäure wurden in 2 ml N-Methyl-2-pyrrolidinon gelöst und bei 0 °C mit 1 ml (1,2 mmol) der 2-Chlor-4-fluorbenzoylisocyanat-Dichlormethan-lösung, hergestellt in Stufe a, versetzt. Der entstandene Niederschlag wurde abgesaugt. Das Rohgemisch (500 mg) wurde mittels Säulenchromatographie (Dichlormethan/Methanol = 98/2 bis 93/7) gereinigt. Man erhielt 80 mg (25 %) des gewünschten Produktes.

### c) 3-[3-(2-Chloro-4-fluoro-benzoyl)-ureido]-4-methoxycarbonylamino-benzoesäure

28 mg (0,08 mmol) 4-Amino-3-[3-(2-chloro-4-fluoro-benzoyl)-ureido]-benzoesäure wurden in 0,5 ml N-Methyl-2-pyrrolidinon gelöst und mit 0,02 ml (0,24 mmol) Pyridin und 0,007 ml Chlorameisensäuremethylester 4 Stunden bei Raumtemperatur gerührt. Man gab Wasser und Essigsäure hinzu und saugte den entstandenen Niederschlag ab. Man erhielt 18 mg (55 %) des gewünschten Produktes.
Smp: Zersetzung > 400°C

### Beispiel 54:

### 3-{2-[3-(2-Chloro-4,5-difluoro-benzoyl)-ureido]-4-[3-(2-trifluoromethyl-phenyl)-ureido]-phenyl}-acrylsäuremethylester

### a) 3-{2-[3-(2-Chloro-4,5-difluoro-benzoyl)-ureido]-4-nitro-phenyl}-acrylsäuremethylester

4,5 g (20,3 mmol) 3-(2-Amino-4-nitro-phenyl)-acrylsäuremethylester wurden mit 4,41 g (20,3 mmol) 2-Chlor-4,5-difluorbenzoylisocyanat (Beispiel 1 a) in 50 ml Acetonitril bei 50 °C für eine Stunde gerührt. Anschließend wurde das Reaktionsgemisch eingeengt, der Rückstand mit Diethylether verrührt und der entstandenen Feststoff abgesaugt. Man erhielt 8,5 g (95 %) des gewünschten Produktes.

### b) 3-{4-Amino-2-[3-(2-chloro-4,5-difluoro-benzoyl)-ureido]-phenyl}-acrylsäuremethylester

8,5 g (19,3 mmol) 3-{2-[3-(2-Chloro-4,5-difluoro-benzoyl)-ureido]-4-nitro-phenyl}-acrylsäuremethylester wurden in 60 ml eines Gemisches von Eisessig und konzentrierter Salzsäure (10:1) suspendiert und auf 70°C erhitzt. Anschließend wurden 8,85 g (135,3 mmol) Zinkpulver zugesetzt. Nach 30 Minuten wurde abgekühlt, der Feststoff abgesaugt und das Filtrat eingeengt. Der Rückstand wurde in Essigsäureethylester aufgenommen und mit 10 %iger Natriumhydrogenkarbonat Lösung gewaschen. Die organische Phase wurde getrocknet und eingeengt. Man erhielt 7,9 g (100 %) des gewünschten Produktes.

### c) 3-{2-[3-(2-Chloro-4,5-difluoro-benzoyl)-ureido]-4-[3-(2-trifluoromethyl-phenyl)-ureido]-phenyl}-acrylsäuremethylester

100 mg (0,24 mmol) 3-{4-Amino-2-[3-(2-chloro-4,5-difluoro-benzoyl)-ureido]-phenyl}-acrylsäuremethylester wurden in 1 ml Acetonitril gelöst und mit 2-Trifluor-methylphenyl-isocyanat versetzt und 14 Stunden bei 60 °C geschüttelt. Der entstandene Niederschlag wurde abgesaugt und man erhielt 16 mg (11 %) des gewünschten Produktes.

### Beispiel 110:

### (E)-3-[2-[3-(2-Chloro-4,5-difluoro-benzoyl)-ureido]-4-(4-chloro-phenoxycarbonylamino)-phenyl]-acrylsäuremethylester

100 mg (0,24 mmol) 3-{4-Amino-2-[3-(2-chloro-4,5-difluoro-benzoyl)-ureido]-phenyl}-acrylsäuremethylester (Beispiel 54 b) wurden in 2 ml Dimethylformamid mit Kaliumkarbonat und Chlorameisensäure-4-chlorphenylester umgesetzt. Nach 4 Stunden wurde der entstandene Niederschlag abgesaugt. Nach präparativer HPLC (Säule: Waters Xterra ^{™}MS C₁₈, 5 µm, 30x100 mm, Laufmittel: A: H₂O + 0,2 % Trifluoressigsäure, B: Acetonitril, Gradient: 2,5 Minuten 90 % A / 10 % B bis 17,5 Minuten 10 % A / 90 % B) erhielt man 12 mg (9 %) des gewünschten Produktes.

### Beispiel 132:

### 1-(2-Chloro-4,5-difluoro-benzoyl)-3-(6-methoxy-2,4-dioxo-1,2,3,4-tetrahydro-quinazolin-7-yl)-harnstoff

### a) N-(4-Methoxy-2-methyl-phenyl)-acetamide

41,1 g (0,3 mol) 4-Methoxy-2-methyl-phenylamine und 37 g (0,5 mol) Dimethylethylamin wurden in 50 ml Tetrahydrofuran gelöst und mit 35,7 g (0,35 mol) Essigsäure-anhydrid unter Rühren versetzt. Dabei erwärmte sich die Lösung zum Sieden. Man rührte 1 Stunde bei Raumtemperatur und kühlte auf 0 °C als. Der entstandene Niederschlag wurde abgesaugt und mehrfach mit wenig kaltem Tetrahydrofuran gewaschen und getrocknet. Man erhielt 40 g (75 %) farblose Kristalle des gewünschten Produktes.

### b) N-(4-Methoxy-2-methyl-5-nitro-phenyl)-acetamide

34 g (0,19 mol) N-(4-Methoxy-2-methyl-phenyl)-acetamide wurden bei -10 bis -15 °C in kleinen Portionen zu einer Mischung aus 40 ml Eisessig und 70 ml rauchende Salpetersäure gegeben. Dabei wurde so portioniert, dass die Temperatur nicht über - 10 °C anstieg. Anschließend wurde das Reaktionsgemisch auf Eis gegossen. Der entstandene Niederschlag wurde abgesaugt und mit Wasser, Ethanol und Diethylether gewaschen. Man erhielt 22,5 g (53 %) des gewünschten Produktes.

### c) 2-Acetylamino-5-methoxy-4-nitro-benzoesäure

11,2 g (50 mmol) N-(4-Methoxy-2-methyl-5-nitro-phenyl)-acetamide und 8,5 g (62,5 mmol) wasserfreies Magnesiumsulfat wurden in 500 ml Wasser suspendiert und auf 85 °C erwärmt. Innerhalb von 30 Minuten wurde eine Lösung aus 21,8 g (138 mmol) Kaliumpermanganat in 250 ml Wasser zugetropft. Man rührte 3 Stunden bei 85 °C und filtrierte das heiße Reaktionsgemisch vom Braunstein ab. Dieser wurde dreimal mit jeweils 100 ml Wasser ausgekocht. Die vereinigten wässrigen Phasen wurden nochmals heiß filtriert und im Vakuum auf ca. 150 ml eingeengt. Der Rückstand wurde mit konzentrierter Salzsäure auf pH 1-2 angesäuert und auf 0 °C abgekühlt. Das entstandene Produkt wurde abgesaugt, mit Wasser und Diethylether gewaschen, getrocknet und ohne weitere Reinigung in der nächsten Stufe umgesetzt.

### d) 2-Amino-5-methoxy-4-nitro-benzoesäure

7,5 g 2-Acetylamino-5-methoxy-4-nitro-benzoesäure (Rohgemisches aus Stufe c) wurden in 50 ml Wasser und 20 ml konzentrierter Salzsäure für 3 Stunden zum Rückfluss erhitzt. Das Reaktionsgemisch wurde im Vakuum eingeengt und säulenchromatographisch (Kieselgel, Dichlormethan/Isopropanol = 9/1) gereinigt. Man erhielt 3,1 g (50 %) des gewünschten Produktes.

### e) 6-Methoxy-7-nitro-1H-benzo[d][1,3]oxazine-2,4-dione

2,0 g (9,4 mmol) 2-Amino-5-methoxy-4-nitro-benzoesäure wurden in 20 ml Chloroform und 10 ml Tetrahydrofuran gelöst und mit 20 ml 20 %iger Phosgen Lösung (1,8 M in Toluol) versetzt. Nach 3 Stunden am Rückfluss wurden bei 60 °C erneut 10 ml Phosgen Lösung zugegeben und weitere 12 Stunden bei 60°C gerührt. Das Phosgen wurde abdestilliert und der Rückstand im Vakuum nach mehrmaliger Zugabe von Toluol eingeengt. Man erhielt 2,2 g (100 %) des gewünschten Produktes.

### f) 2-Amino-5-methoxy-4-nitro-benzamide

476 mg (2 mmol) 6-Methoxy-7-nitro-1H-benzo[d][1,3]oxazine-2,4-dione und 1,5 g (20 mmol) Ammoniumacetat wurden in 20 ml Essigsäure gelöst und 3 Stunden auf 105 °C erhitzt. Anschließend wurde das Reaktionsgemisch auf Eiswasser gegossen und langsam mit festem Natriumhydrogenkarbonat auf pH 7 gebracht. Die wässrige Phase wurde mit Essigsäureethylester extrahiert. Die organische Phase wurde mit Wasser gewaschen, getrocknet und im Vakuum eingeengt. Das Rohprodukt wurde mit Diethylether verrieben und der entstandene Niederschlag wurde abgesaugt. Man erhielt 285 mg (68 %) des gewünschten Produktes.

### g) 6-Methoxy-7-nitro-1 H-quinazoline-2,4-dione

108 mg (0,5 mmol) 2-Amino-5-methoxy-4-nitro-benzamide wurden in 5 ml Tetrahydrofuran und 5 ml Chloroform gelöst und mit Oxalylchlorid (Lösung in Toluol) versetzt. Man rührte 5 Stunden bei 60 °C und engte anschließend im Vakuum ein. Nach Zugabe von Toluol wurde erneut eingeengt. Man erhielt 120 mg (100 %) des gewünschten Produktes.

### h) 7-Amino-6-methoxy-1 H-quinazoline-2,4-dione

120 mg (0,5 mmol) 6-Methoxy-7-nitro-1 H-quinazoline-2,4-dione wurden in einem Gemisch von 5 ml Tetrahydrofuran, 5 ml Methanol und 5 ml Essigsäure aufgenommen und 3 Stunden bei Raumtemperatur unter Palladium Katalyse hydriert. Die Reaktionslösung wurde erwärmt um ausgefallenes Produkt zu lösen und warm vom Katalysator abfiltriert. Das Filtrat wurde eingeengt und nach Zugabe von Toluol erneut eingeengt. Man erhielt 100 mg (100 %) des gewünschten Produktes.

### i) 1-(2-Chloro-4,5-difluoro-benzoyl)-3-(6-methoxy-2,4-dioxo-1,2,3,4-tetrahydro-quinazolin-7- yl)-harnstoff

100 mg (0,53 mmol) 7-Amino-6-methoxy-1H-quinazoline-2,4-dione wurden in 20 ml Acetonitril und 2 ml N-Methyl-2-pyrrolidinon suspendiert und mit 200 mg (0,97 mmol) 2-Chlor-4,5-difluorbenzoylisocyanat (Beispiel 1 a) versetzt. Das Reaktionsgemisch wurde zum Sieden erhitzt, anschließend mit Methanol gequenscht und im Vakuum eingeengt. Der Rückstand wurde mit Acetonitril verrührt und der entstandene Niederschlag wurde abfiltriert. Man erhielt 64 mg (30 %) des gewünschten Produktes.

## Patentansprüche

1. Verbindungen der Formel la 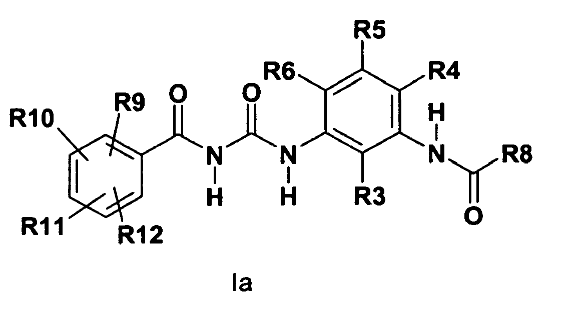 worin bedeuten
R9, R10, R11 unabhängig von einander F oder Cl;
R12 H;
R13 unabhängig voneinander H oder (C₁-C₆)-Alkyl;
R3, R4, R5 unabhängig voneinander H, COOR13;
R6 Cl, OCF₃, COOR13, N(R14)(R15), (C₂-C₆)-Alkenyl, O-(C₁-C₁₀)-Alkyl, wobei Alkyl, Alkenyl mehrfach mit F, COOR13 oder CON(R14)(R15) substituiert sein können;
R14, R15 (C₁-C₆)-Alkyl, wobei Alkyl mit N(R13)₂ substituiert sein kann;
oder die Reste R14 und R15 mit dem Stickstoffatom an das sie gebunden sind einem 5 gliedrigen, gesättigten heterocyclischen Ring bilden;
R8 N(R18)(R19) oder OR20;
R18, R19 unabhängig voneinander H, (C₁-C₁₀)-Alkyl, (C₂-C₁₀)-Alkenyl, wobei Alkyl durch COOR13, N(R13)₂ oder Phenyl substituiert sein kann, (C₃-C₇)-Cycloalkyl oder (C₆-C₁₀)-Aryl, wobei Aryl mehrfach mit F, Cl, CN, (C₁-C₆)-Alkyl, O-(C₁-C₆)-Alkyl, CO-(C₁-C₆)-Alkyl, wobei Alkyl mehrfach durch F substituiert sein kann, O-Phenyl, Phenyl, Pyridyl oder COOR13 substituiert sein kann;
R20 (C₁-C₁₀)-Alkyl, (C₂-C₁₀)-Alkenyl, (C₂-C₁₀)-Alkinyl oder Phenyl, wobei Phenyl mit Cl oder (C₁-C₆)-Alkyl substituiert sein kann;
sowie deren physiologisch verträgliche Salze.

2. Arzneimittel enthaltend eine oder mehrere der Verbindungen gemäß Anspruch 1.

3. Arzneimittel enthaltend eine oder mehrere der Verbindungen gemäß Anspruch 1 und mindestens einen weiteren Wirkstoff.

4. Arzneimittel, gemäß Anspruch 3, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

5. Verwendung der Verbindungen gemäß Anspruch 1 zur Herstellung eines Medikamentes zur Blutzuckersenkung.

6. Verwendung der Verbindungen gemäß Anspruch 1 zur Herstellung eines Medikamentes zur Behandlung des Typ II Diabetes.

7. Verwendung der Verbindungen gemäß Anspruch 1 zur Herstellung eines Medikamentes zur Behandlung von Lipid- und Kohlenhydratstoffwechselstörungen.

8. Verwendung der gemäß Anspruch 1 zur Herstellung eines Medikaments zur Behandlung arteriosklerotischer Erscheinungen.

9. Verwendung der Verbindungen gemäß Anspruch 1 zur Herstellung eines Medikaments zur Behandlung von Insulin Resistenz.

10. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

## Claims

1. A compound of the formula Ia 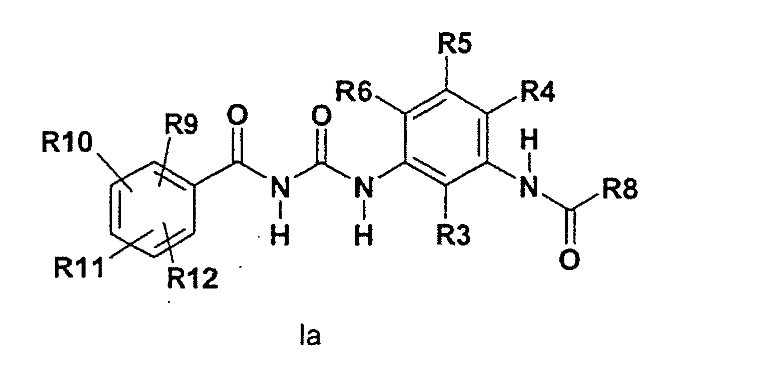 in which the meanings are
R9, R10, R11 independently of one another F or Cl;
R12 H;
R13 independently of one another H or (C₁-C₆)-alkyl;
R3, R4, R5 independently of one another H, COOR13;
R6 Cl, OCF₃, COOR13, N(R14) (R15), (C₂-C₆) -alkenyl, O-(C₁-C₁₀)-alkyl, where alkyl, alkenyl may be substituted more than once by F, COOR13 or CON(R14) (R15) ;
R14, R15 (C₁-C₆)-alkyl, where alkyl may be substituted by N(R13)₂;
or the radicals R14 and R15 form with the nitrogen atom to which they are bonded a 5-membered, saturated heterocyclic ring;
R8 N(R18) (R19) or OR20;
R18, R19 independently of one another H, (C₁-C₁₀)-alkyl, (C₂-C₁₀) -alkenyl, where alkyl may be substituted by COOR13, N(R13)₂ or phenyl, or (C₃-C₇)-cycloalkyl or (C₆-C₁₀)-aryl, where aryl may be substituted more than once by F, Cl, CN, (C₁-C₆) -alkyl, O-(C₁-C₆)-alkyl, CO-(C₁-C₆)-alkyl, where alkyl may be substituted more than once by F, or O-phenyl, phenyl, pyridyl or COOR13;
R20 (C₁-C₁₀)-alkyl, (C₂-C₁₀)-alkenyl, (C₂-C₁₀)-alkynyl or phenyl, where phenyl may be substituted by Cl or (C₁-C₆) -alkyl;
and its physiologically tolerated salts.

2. A medicament comprising one or more of the compounds as claimed in claim 1

3. A medicament comprising one or more of the compounds as claimed in claim 1 and at least one other active ingredient.

4. A medicament as claimed in claim 3, wherein the other active ingredient comprises one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

5. The use of the compounds as claimed in claim 1 for producing a medicament for reducing blood glucose.

6. The use of the compounds as claimed in claim 1 for producing a medicament for the treatment of type II diabetes.

7. The use of the compounds as claimed in claim 1 for producing a medicament for the treatment of disturbances of lipid and carbohydrate metabolism.

8. The use of the compounds as claimed in claim 1 for producing a medicament for the treatment of arteriosclerotic manifestations.

9. The use of the compounds as claimed in claim 1 for producing a medicament for the treatment of insulin resistance.

10. A process for producing a medicament comprising one or more of the compounds as claimed in claim 1, which comprises mixing the active ingredient with a pharmaceutically suitable carrier and converting this mixture into a form suitable for administration.

## Revendications

1. Composés de formule Ia 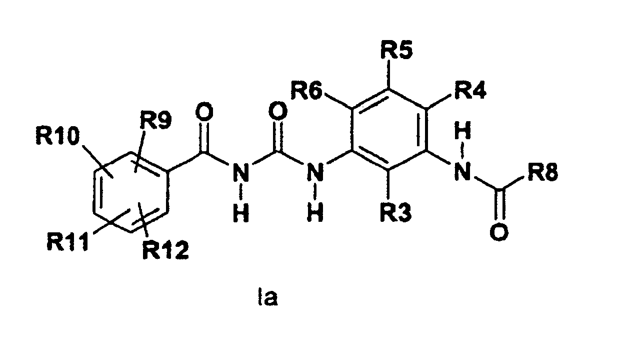 dans laquelle
R9, R10, R11 représentent, chacun indépendamment, F ou Cl ;
R12 représente H ;
R13 représente, chaque fois indépendamment, H ou un groupe alkyle en C₁-C₆ ;
R3, R4, R5 représentent, chacun indépendamment, H, COOR13 ;
R6 représente Cl, OCF₃, COOR13, N(R14)(R15), alcényle en C₂-C₆, O-alkyle(C₁-C₁₀), les groupes alkyle, alcényle pouvant être plusieurs fois substitués par F, COOR13 ou CON(R14)(R15) ;
R14, R15 représentent un groupe alkyle en C₁-C₆, le groupe alkyle pouvant être substitué par N(R13)₂ ;
ou les radicaux R14 et R15 forment avec l'atome d'azote auquel ils sont liés un cycle hétérocyclique saturé à 5 chaînons ;
R8 représente N(R18)(R19) ou OR20 ;
R18, R19 représentent, indépendamment l'un de l'autre, H, un groupe alkyle en C₁-C₁₀, alcényle en C₂-C₁₀, le groupe alkyle pouvant être substitué par COOR13, N(R13)₂ ou phényle, un groupe cycloalkyle en C₃-C₇ ou aryle en C₆-C₁₀, le groupe aryle pouvant être plusieurs fois substitué par F, Cl, CN, alkyle en C₁-C₆, O-alkyle(C₁-C₆), CO-alkyle(C₁-C₆) (le groupe alkyle pouvant être plusieurs fois substitué par F), O-phényle, phényle, pyridyle ou COOR13 ;
R20 représente un groupe alkyle en C₁-C₁₀, alcényle en C₂-C₁₀, alcynyle en C₂-C₁₀ ou phényle, le groupe phényle pouvant être substitué par Cl ou alkyle en C₁-C₆ ;
ainsi que leurs sels physiologiquement acceptables.

2. Médicament contenant un ou plusieurs des composés selon la revendication 1.

3. Médicament contenant un ou plusieurs des composés selon la revendication 1 et au moins une autre substance active.

4. Médicament selon la revendication 3, **caractérisé en ce qu'**il contient comme autre substance active un(e) ou plusieurs antidiabétiques, principes actifs hypoglycémiants, inhibiteurs de HMGCoA-réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption d'acides biliaires, inhibiteurs de CETP, adsorbeurs polymères d'acides biliaires, inducteurs de récepteurs de LDL, inhibiteurs d'ACAT, antioxydants, inhibiteurs de lipoprotéine lipase, inhibiteurs d'ATP-citrate lyase, inhibiteurs de squalène synthétase, antagonistes de lipoprotéine (a), inhibiteurs de lipase, insulines, sulfonylurées, biguanides, méglitinides, thiazolidinediones, inhibiteurs d'α-glucosidase, principes actifs agissant sur le canal potassique ATP-dépendant des cellules bêta, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, agonistes de MSH (hormone simulant les mélanocytes), agonistes de CCK, inhibiteurs de la recapture de sérotonine, composés sérotoninergiques et noradrénergiques mixtes, agonistes de 5HT, agonistes de bombésine, antagonistes de galanine, hormones de croissance, composés libérant l'hormone de croissance, agonistes de TRH, modulateurs de protéine 2 ou 3 découpleurs, agonistes de leptine, agonistes de DA (bromocriptine, doprexine), inhibiteurs de lipase/amylase, modulateurs de PPAR, modulateurs de RXR ou agonistes de TR-β ou amphétamines.

5. Utilisation des composés selon la revendication 1, pour la fabrication d'un médicament destiné à abaisser la glycémie.

6. Utilisation des composés selon la revendication 1, pour la fabrication d'un médicament destiné au traitement du diabète de type II.

7. Utilisation des composés selon la revendication 1, pour la fabrication d'un médicament destiné au traitement de troubles du métabolisme des glucides et des lipides.

8. Utilisation des composés selon la revendication 1, pour la fabrication d'un médicament destiné au traitement de manifestations artérioscléreuses.

9. Utilisation des composés selon la revendication 1, pour la fabrication d'un médicament destiné au traitement de la résistance à l'insuline.

10. Procédé pour la fabrication d'un médicament contenant un ou plusieurs des composés selon la revendication 1, **caractérisé en ce qu'**on mélange le principe actif avec un véhicule pharmaceutiquement convenable et on met ce mélange sous une forme appropriée à l'administration.
